# EUROPÄISCHE PATENTSCHRIFT

(11) **EP 2 668 005 B1**
(45) Veröffentlichungstag und Bekanntmachung des Hinweises auf die Patenterteilung: **03.10.2018**
(21) Anmeldenummer: 11802759.8
(22) Anmeldetag: 27.12.2011
(51) Int. Cl.: B24B 45/00, B24B 23/04

(54) **WERKZEUGSPANNVORRICHTUNG**
TOOL CHUCKING DEVICE
DISPOSITIF DE SERRAGE D'OUTIL

(30) Priorität: 25.01.2011 DE 102011003100
(43) Veröffentlichungstag der Anmeldung: 04.12.2013
(73) Patentinhaber: Robert Bosch GmbH, 70442 Stuttgart (DE)
(72) Erfinder: FANKHAUSER, Marcel, CH-3011 Bern (CH); LUESCHER, Bruno, CH-4800 Zofingen (CH)
(86) Internationale Anmeldenummer: PCT/EP2011/074107
(87) Internationale Veröffentlichungsnummer: WO 2012/100894

(56) Entgegenhaltungen:
- EP-A2- 1 180 416
- DE-U1-202008 001 759
- US-B1- 7 128 641

## Beschreibung

### Stand der Technik

Es sind bereits Werkzeugspannvorrichtungen, insbesondere Oszillationswerkzeugspannvorrichtungen, bekannt, die eine Spanneinheit aufweisen. Die Spanneinheit weist hierbei ein Spannelement zu einem Festspannen eines Bearbeitungswerkzeugs in einer Axialrichtung und eine Bedieneinheit zur Betätigung des Spannelements auf, wie zum Beispiel in den Druckschriften DE 20 2008 001 759 U1 und EP 1 180 416 A2.

### Offenbarung der Erfindung

Die Erfindung geht aus von einer Werkzeugspannvorrichtung, insbesondere von einer Oszillationswerkzeugspannvorrichtung, mit zumindest einer Spanneinheit, die zumindest ein Spannelement zu einem Festspannen eines Bearbeitungswerkzeugs in einer Axialrichtung sowie zumindest eine Bedieneinheit zur Betätigung des Spannelements aufweist.
Es wird vorgeschlagen, dass die Werkzeugspannvorrichtung zumindest eine Übersetzungseinheit umfasst, die dazu vorgesehen ist, in Abhängigkeit zumindest einer Bewegungskomponente eines Bedienelements der Bedieneinheit ein Übersetzungsverhältnis zu verändern. In diesem Zusammenhang soll der Begriff "vorgesehen" speziell ausgestattet und/oder speziell ausgelegt definieren. Unter einer "Spanneinheit" soll hier insbesondere eine Einheit verstanden werden, die ein Bearbeitungswerkzeug mittels eines Formschlusses und/oder mittels eines Kraftschlusses entlang der Axialrichtung sichert, insbesondere an einer Werkzeugaufnahme einer tragbaren Werkzeugmaschine. Bevorzugt wirkt in einem Spannmodus der Spanneinheit eine Spannkraft entlang der Axialrichtung auf das Bearbeitungswerkzeug. Bevorzugt ist das Spannelement der Spanneinheit stiftförmig ausgebildet. Unter einem "stiftförmigen Spannelement" soll hier insbesondere ein Spannelement verstanden werden, das in einem montierten Zustand eine Längserstreckung entlang der Axialrichtung aufweist, die größer ist als eine Quererstreckung des Spannelements entlang einer senkrecht zur Axialrichtung verlaufenden Richtung. Insbesondere ist die Längserstreckung mehr als doppelt so groß als die Querstreckung des Spannelements, bevorzugt mehr als vier Mal so groß und besonders bevorzugt mehr als sechs Mal so groß. Bevorzugt ist das stiftförmige Spannelement zumindest teilweise als Hohlkörper ausgebildet. Besonders bevorzugt weist das Spannelement zumindest zwei als Schenkel ausgebildete Teilbereiche auf, die zumindest teilweise entlang einer zumindest im Wesentlichen senkrecht zur Axialrichtung verlaufenden Richtung relativ zueinander beabstandet angeordnet sind. Vorzugsweise ist das Spannelement verliersicher in einer Hohlwelle der tragbaren Werkzeugmaschine angeordnet. Besonders bevorzugt weist die Spanneinheit einen am Spannelement angeordneten Spannkopf auf. Hierbei umfasst der Spannkopf vorzugsweise zwei relativ zueinander bewegliche Teilbereiche. Die Teilbereiche des Spannkopfs sind bevorzugt jeweils einstückig mit einem der Schenkel des Spannelements ausgebildet. Unter einem "Spannkopf" soll hier insbesondere ein Element verstanden werden, das zumindest eine Spannfläche aufweist, die zum Festspannen des Bearbeitungswerkzeugs in Axialrichtung zumindest an einer Teilfläche des Bearbeitungswerkzeugs anliegt und das Bearbeitungswerkzeug mit einer Spannkraft entlang der Axialrichtung beaufschlagt. Der Begriff "Axialrichtung" soll hier insbesondere eine Richtung definieren, die bevorzugt zumindest im Wesentlichen parallel zu einer Schwenkachse und/oder Rotationsachse einer zum Antrieb des Bearbeitungswerkzeugs vorgesehenen Antriebswelle und/oder Spindel einer tragbaren Werkzeugmaschine verläuft. Unter "im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist.

Unter einer "Bedieneinheit" soll hier insbesondere eine Einheit verstanden werden, die zumindest ein Bedienelement aufweist, das direkt von einem Bediener betätigbar ist, und die dazu vorgesehen ist, durch eine Betätigung und/oder durch eine Eingabe von Parametern einen Prozess und/oder einen Zustand einer mit der Bedieneinheit gekoppelten Einheit zu beeinflussen und/oder zu ändern. Der Begriff "Übersetzungseinheit" soll hier insbesondere eine Einheit definieren, die dazu vorgesehen ist, zumindest einen Wert einer physikalischen Größe, wie beispielsweise eine Drehzahl, ein Drehmoment, eine Kraft usw., in einen anderen Wert derselben physikalischen Größe zu übersetzen, wobei beide Werte in einem festgelegten Verhältnis, insbesondere in einem konstruktiv festgelegten Verhältnis, zueinander stehen. Bevorzugt wird die Übersetzungseinheit von einer mechanischen Übersetzungseinheit gebildet. Es ist jedoch auch denkbar, dass die Übersetzungseinheit auf eine andere, einem Fachmann als sinnvoll erscheinenden Art und Weise ausgestaltet ist.

Unter einer "Bewegungskomponente" soll hier insbesondere eine Komponente einer Bewegungsgröße verstanden werden, die eine Bewegung mathematisch definiert, wie beispielsweise ein Weg, eine Geschwindigkeit, ein Winkel usw. Bevorzugt wird die Bewegungskomponente von einem Winkel, insbesondere von einem Öffnungswinkel, gebildet, den das Bedienelement der Bedieneinheit bei einer Bewegung ausgehend von einer Ausgangsstellung überstreicht. Der Begriff "Ausgangsstellung" soll hier insbesondere eine Position des Bedienelements definieren, in der eine Krafteinwirkung des Bedienelements auf die Übersetzungseinheit und/oder die Spanneinheit zur Betätigung der Spanneinheit aufgehoben und/oder unterbunden ist. Bevorzugt wird durch die Übersetzungseinheit in Abhängigkeit eines von dem Bedienelement überstrichenen Öffnungswinkels ein Übersetzungsverhältnis zwischen einer zurückgelegten Strecke des Bedienelements, insbesondere um eine zumindest im Wesentlichen parallel zur Axialrichtung verlaufenden Rotationsachse, und einer von dem Spannelement zurückgelegten Strecke entlang der Axialrichtung geändert. Der Begriff "Übersetzungsverhältnis" soll hier insbesondere ein Verhältnis von mittels der Übersetzungseinheit änderbaren physikalischen Werten relativ zueinander definieren, wobei beide physikalischen Werte von Null abweichend sind, insbesondere ist das Verhältnis selbst ebenfalls von einem Wert von Null und/oder Unendlich abweichend. Mittels der erfindungsgemäßen Ausgestaltung der Werkzeugspannvorrichtung kann vorteilhaft eine Bewegungsumsetzung zur Betätigung der Spanneinheit beeinflusst werden. Es kann ferner vorteilhaft ein an einen Anwendungsfall angepasstes Übersetzungsverhältnis erreicht werden. Somit kann besonders vorteilhaft ein hoher Bedienkomfort erreicht werden.

Des Weiteren wird vorgeschlagen, dass die Übersetzungseinheit zumindest eine Steuerkurve umfasst, die, entlang eines Verlaufs der Steuerkurve in zumindest zwei verschiedenen Punkten der Steuerkurve betrachtet, zueinander verschiedene Steigungen in Axialrichtung aufweist. Besonders bevorzugt weisen die unterschiedlichen Steigungen der Steuerkurve in den zwei verschiedenen Punkten entlang eines Verlaufs der Steuerkurve jeweils einen von Null abweichenden Wert auf. Unter einer "Steuerkurve" soll hier insbesondere eine geometrische Gestalt verstanden werden, die dazu vorgesehen ist, eine Bewegungsform in eine andere Bewegungsform umzuwandeln und/oder die dazu vorgesehen ist, infolge einer Bewegung, insbesondere einer Bewegung der Steuerkurve um eine Achse, ein Bauteil anzusteuern, das infolge der Bewegung eine durch die geometrische Gestalt vorbestimmte Bewegung ausführt. Bevorzugt ist die Steuerkurve dazu vorgesehen, eine Drehbewegung in einer translatorische Bewegung umzuwandeln. Der Begriff "Steigung" soll hier insbesondere ein Maß für eine Steilheit der Steuerkurve, insbesondere betrachtet in einer Ebene und/oder einer Projektionsebene, definieren. Bevorzugt wird die Steigung von einer mathematisch definierten Steigung gebildet, die mittels eines Differenzquotienten und/oder mittels einer Differentialgleichung in jedem beliebigen Punkt der Steuerkurve ermittelt werden kann. Bevorzugt ist die Steuerkurve an einer der Bedieneinheit zugewandten Seite des Spannelements am Spannelement angeordnet. Besonders bevorzugt ist die Steuerkurve einstückig mit dem Spannelement ausgebildet. Mittels der Steuerkurve kann konstruktiv einfach eine Bewegungsumsetzung realisiert werden. Ferner kann mittels der sich ändernden Steigung der Steuerkurve vorteilhaft eine an einen Anwendungsfall angepasste Bewegungsumsetzung erreicht werden. Des Weiteren kann besonders vorteilhaft eine von einem Bediener aufzubringende Betätigungskraft zur Betätigung des Spannelements mittels der Bedieneinheit beeinflusst werden, insbesondere kann vorteilhaft ein Verlauf der Betätigungskraft, während einer Betätigung betrachtet, in Abhängigkeit eines Öffnungswinkels des Bedienelements verändert werden.

Vorteilhafterweise weist die Übersetzungseinheit zumindest ein Abtastelement auf, das dazu vorgesehen ist, das Spannelement in Abhängigkeit des Verlaufs der Steuerkurve entlang der Axialrichtung zu bewegen. Unter einem "Abtastelement" soll hier insbesondere ein Element verstanden werden, das den Verlauf der Steuerkurve abtastet, insbesondere mechanisch abtastet, und infolge des Verlaufs der Steuerkurve ein Bauteil ansteuert, das eine vom Verlauf der Steuerkurve abhängige Bewegung ausführt. Bevorzugt liegt das Abtastelement zumindest in einem Betriebszustand auf der Steuerkurve auf. Es kann vorteilhaft eine von dem Verlauf der Steuerkurve abhängige Bewegung des Spannelements erreicht werden.

Vorteilhafterweise ist das Abtastelement als Bolzen ausgebildet. Unter einem "Bolzen" soll hier insbesondere ein Element verstanden werden, das eine Längserstreckung aufweist, die größer ist als eine senkrecht zur Längserstreckung verlaufende Quererstreckung. Bevorzugt ist der Bolzen zylinderförmig ausgebildet. Besonders bevorzugt ist der Bolzen rotationssymmetrisch um zumindest eine Achse ausgebildet. Vorzugsweise ist der Bolzen aus einem Vollmaterial gebildet. Es ist jedoch auch denkbar, dass das Abtastelement eine andere, einem Fachmann als sinnvolle erscheinende Ausgestaltung aufweist. Es kann konstruktiv einfach ein Abtastelement erreicht werden.

Ferner wird vorgeschlagen, dass der Bolzen eine Längserstreckung aufweist, die in einem montierten Zustand entlang einer sich zumindest im Wesentlichen senkrecht zur Axialrichtung erstreckenden Richtung verläuft. Es ist jedoch auch denkbar, dass der Bolzen eine Längserstreckung aufweist, die in einem montierten Zustand zum Abtasten eines Verlaufs der Steuerkurve entlang einer anderen, einem Fachmann als sinnvoll erscheinenden Richtung verläuft. Es kann konstruktiv einfach eine Auflagefläche und/oder eine Anlagefläche zwischen dem Bolzen und der Steuerkurve erreicht werden.

Zudem wird vorgeschlagen, dass die Werkzeugspannvorrichtung zumindest eine Entkopplungseinheit umfasst, die dazu vorgesehen ist, die Bedieneinheit in zumindest einem Betriebsmodus von einer Bewegung des Spannelements zu entkoppeln. Unter einer "Entkopplungseinheit" soll hier insbesondere eine Einheit verstanden werden, die zumindest einen Mechanismus und/oder zumindest ein Bauteil aufweist, das eine Entkopplung, insbesondere eine Entkopplung von Bewegungen, zwischen zumindest zwei Elementen bewirkt. Bevorzugt ist die Entkopplungseinheit dazu vorgesehen, die Bedieneinheit in zumindest einem Betriebsmodus von einer oszillierenden Bewegung des Spannelements um eine zumindest im Wesentlichen parallel zur Axialrichtung verlaufende Schwenkachse des Spannelements zu entkoppeln. Es kann vorteilhaft eine Schonung von Bauteilen in zumindest einem Betriebsmodus erreicht werden. Somit kann vorteilhaft eine lange Lebensdauer der Werkzeugspannvorrichtung erreicht werden. Vorzugsweise weist die Entkopplungseinheit zumindest ein Anschlagelement auf, das dazu vorgesehen ist, eine Bewegung des Spannelements entlang der Axialrichtung in Richtung der Bedieneinheit zu begrenzen. Bevorzugt ist das Anschlagelement infolge der Begrenzung der Bewegung des Spannelements entlang der Axialrichtung dazu vorgesehen, zumindest in einem Betriebszustand einen Abstand zwischen dem Abtastelement und der Steuerkurve entlang einer senkrecht zur Axialrichtung verlaufenden Richtung zu gewährleisten. Somit kann vorteilhaft eine Übertragung einer Bewegung des Spannelements über die Steuerkurve und über das Abtastelement in zumindest einem Betriebszustand an das Bedienelement der Bedieneinheit verhindert werden.

Das Bedienelement der Bedieneinheit ist als Bedienhebel ausgebildet, der schwenkbar um eine parallel zur Axialrichtung verlaufende Schwenkachse gelagert ist. Der Bedienhebel ist schwenkbar und/oder drehbar in einem Werkzeugmaschinengehäuse der tragbaren Werkzeugmaschine gelagert. Bevorzugt ist der Bedienhebel als einseitiger Hebel ausgebildet, bei dem Betätigungskräfte auf einer Seite des Bedienhebels eingeleitet werden. Es ist jedoch auch denkbar, dass der Bedienhebel in einer anderen, einem Fachmann als sinnvoll erscheinenden Art und Weise ausgestaltet ist. Es kann vorteilhaft eine Betätigungskraft eines Bedieners zur Betätigung des Spannelements mittels des Bedienhebels aufgebracht werden. Vorteilhafterweise umfasst die Bedieneinheit zumindest ein Abtastaufnahmeelement zur Aufnahme eines Abtastelements der Bedieneinheit, das zumindest drehfest mit dem Bedienhebel verbunden ist. Unter "drehfest verbunden" soll hier insbesondere eine Verbindung verstanden werden, die ein Drehmoment und/oder eine Drehbewegung unverändert überträgt. Bevorzugt ist das Abtastaufnahmeelement mittels einer Schraubverbindung drehfest mit dem Bedienhebel verbunden. Es ist jedoch auch denkbar, dass das Abtastaufnahmeelement mittels einer anderen, einem Fachmann als sinnvoll erscheinenden Verbindungsart, wie beispielsweise stoffschlüssig und/oder formschlüssig, mit dem Bedienhebel verbunden ist. Bevorzugt weist das Abtastaufnahmeelement zumindest eine Ausnehmung auf, in der das Abtastelement in einem montierten Zustand angeordnet ist. Es kann vorteilhaft eine Lagerung des Abtastelements erreicht werden. Ferner kann konstruktiv einfach das Abtastelement infolge der Bewegung des Bedienhebels den Verlauf der Steuerkurve abtasten und somit das Spannelement entlang der Axialrichtung bewegen.

Ferner geht die Erfindung aus von einer tragbaren Werkzeugmaschine, insbesondere von einer tragbaren Werkzeugmaschine mit einer oszillierend antreibbaren Spindel, mit zumindest einer erfindungsgemäßen Werkzeugspannvorrichtung. Unter einer "tragbaren Werkzeugmaschine" soll hier insbesondere eine Werkzeugmaschine, insbesondere eine Handwerkzeugmaschine, verstanden werden, die von einem Bediener transportmaschinenlos transportiert werden kann. Die tragbare Werkzeugmaschine weist insbesondere eine Masse auf, die kleiner ist als 40 kg, bevorzugt kleiner als 10 kg und besonders bevorzugt kleiner als 5 kg. Es kann vorteilhaft ein hoher Bedienkomfort für einen Bediener der Werkzeugmaschine erreicht werden.

Die erfindungsgemäße Werkzeugspannvorrichtung soll hierbei nicht auf die oben beschriebene Anwendung und Ausführungsform beschränkt sein. Insbesondere kann die erfindungsgemäße Werkzeugspannvorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnung

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine erfindungsgemäße Werkzeugmaschine mit einer erfindungsgemäßen Werkzeugspannvorrichtung in einer schematischen Darstellung,
- Fig. 2: eine Detailansicht einer Übersetzungseinheit der erfindungsgemäßen Werkzeugspannvorrichtung in einer schematischen Darstellung,
- Fig. 3: eine Detailansicht einer Steuerkurve der Übersetzungseinheit der erfindungsgemäßen Werkzeugspannvorrichtung in einer schematischen Darstellung,
- Fig. 4: eine Schnittansicht der erfindungsgemäßen Werkzeugspannvorrichtung in einer schematischen Darstellung,
- Fig. 5: eine weitere Schnittansicht der erfindungsgemäßen Werkzeugspannvorrichtung in einer schematischen Darstellung,
- Fig. 6: ein Diagramm eines Verlaufs einer Betätigungskraft einer Bedieneinheit der erfindungsgemäßen Werkzeugspannvorrichtung in einer schematischen Darstellung und
- Fig. 7: ein Diagramm eines Verlaufs einer Bewegungsstrecke eines Spannelements einer Spanneinheit der erfindungsgemäßen Werkzeugspannvorrichtung in einer schematischen Darstellung.

### Beschreibung des Ausführungsbeispiels

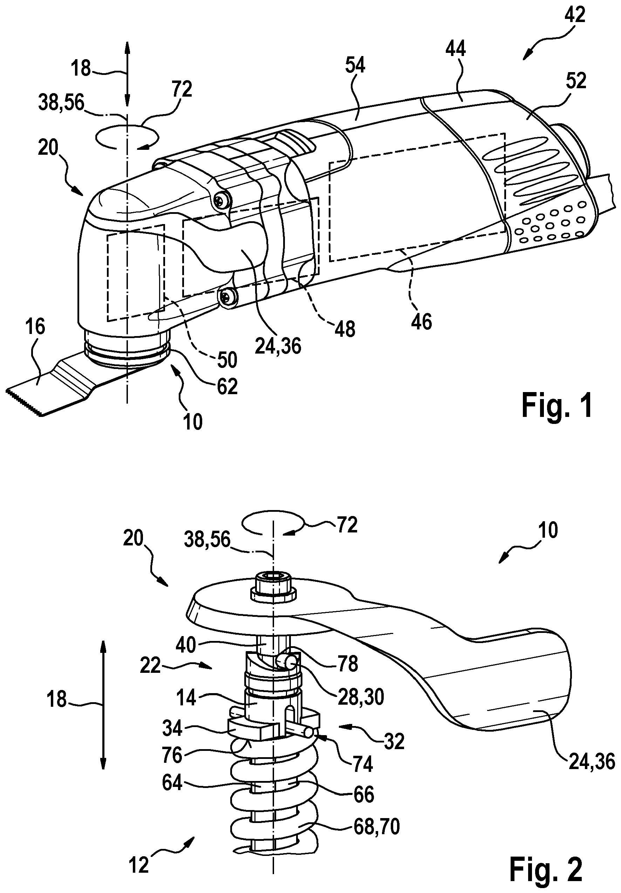

Figur 1 zeigt eine elektrisch betriebene tragbare Werkzeugmaschine 42 mit einer Werkzeugspannvorrichtung 10. Die tragbare Werkzeugmaschine 42 umfasst ein Werkzeugmaschinengehäuse 44, das eine Elektromotoreinheit 46, eine Getriebeeinheit 48 und eine Abtriebseinheit 50 der tragbaren Werkzeugmaschine 42 umschließt. Das Werkzeugmaschinengehäuse 44 umfasst hierbei zwei Gehäusehalbschalen 52, 54, die lösbar entlang einer durch eine Axialrichtung 18 verlaufenden Ebene miteinander verbunden sind. Es ist jedoch auch denkbar, dass das Werkzeugmaschinengehäuse 44 zwei oder mehr topfförmige Gehäuseteile aufweist, die lösbar miteinander verbindbar sind. Die Axialrichtung 18 verläuft entlang und/oder parallel zu einer Rotationsachse 56 einer als Spindel 58 ausgebildeten Hohlwelle 60 der Abtriebseinheit 50 (Figur 2). Die Hohlwelle 60 ist dazu vorgesehen, in einem montierten Zustand ein Bearbeitungswerkzeug 16 oszillierend anzutreiben. Ein oszillierender Antrieb des Bearbeitungswerkzeugs 16 erfolgt hierbei auf eine einem Fachmann bereits bekannte Art und Weise, wie beispielsweise mittels eines exzentrisch auf einer Antriebswelle der Elektromotoreinheit 46 angeordneten Zapfens (hier nicht näher dargestellt) der Getriebeeinheit 48, der mittels einer Schwinge und einer Schwinghülse (hier nicht näher dargestellt) der Getriebeeinheit 48 die Hohlwelle 60 in einem Betrieb der tragbaren Werkzeugmaschine 42 antreibt. Somit ist die als Spindel 58 ausgebildete Hohlwelle 60 oszillierend antreibbar. Das Bearbeitungswerkzeug 16 ist zur spanenden Bearbeitung von Werkstücken an einer Werkzeugaufnahme 62 der Abtriebseinheit 50 befestigbar. Die Werkzeugaufnahme 62 ist mittels einer formschlüssigen und/oder kraftschlüssigen Verbindung drehfest mit der Hohlwelle 60 verbunden. Es ist jedoch auch denkbar, dass die Werkzeugaufnahme 62 einstückig mit der Hohlwelle 60 ausgebildet ist. Es kann eine Schwenkbewegung der Hohlwelle 60 auf die Werkzeugaufnahme 62 übertragen werden.

Figur 2 zeigt eine Detailansicht der Werkzeugspannvorrichtung 10. Die Werkzeugspannvorrichtung 10 umfasst eine Spanneinheit 12, die ein Spannelement 14 zu einem Festspannen des Bearbeitungswerkzeugs 16 in Axialrichtung 18 sowie eine Bedieneinheit 20 zur Betätigung des Spannelements 14 aufweist. Das Spannelement 14 ist stiftförmig ausgebildet. Ferner ist das Spannelement 14 beweglich in der Hohlwelle 60 angeordnet. Hierbei erstreckt sich das Spannelement 14 entlang der Axialrichtung 18 durch die Hohlwelle 60 hindurch. Somit ist das Spannelement 14 in einem montierten Zustand in der Hohlwelle 60 angeordnet. Des Weiteren weist das Spannelement 14 zwei Schenkel 64, 66 auf, die sich in einem montierten Zustand des Spannelements 14 zumindest im Wesentlichen entlang der Axialrichtung 18 erstrecken. Die Schenkel 64, 66 sind einstückig mit dem Spannelement 14 ausgebildet. Ferner weisen die Schenkel 64, 66 eine geringe Materialstärke, entlang einer senkrecht zur Axialrichtung 18 verlaufenden Richtung betrachtet, zur Ermöglichung einer Auslenkung der Schenkel 64, 66 auf. Somit sind die Schenkel 64, 66 infolge von Materialeigenschaften und/oder einer geometrischen Form der Schenkel 64, 66 relativ zueinander beweglich am Spannelement 14 angeordnet. Die Schenkel 64, 66 sind hierbei federnd am Spannelement 14 angeordnet. Des Weiteren sind die Schenkel 64, 66 entlang der senkrecht zur Axialrichtung 18 verlaufenden Richtung relativ zueinander beabstandet angeordnet. Die Schenkel 64, 66 können sich infolge der federnden Anordnung am Spannelement 14 und dem relativen Abstand zueinander entlang der senkrecht zur Axialrichtung 18 verlaufenden Richtung relativ zueinander bewegen.

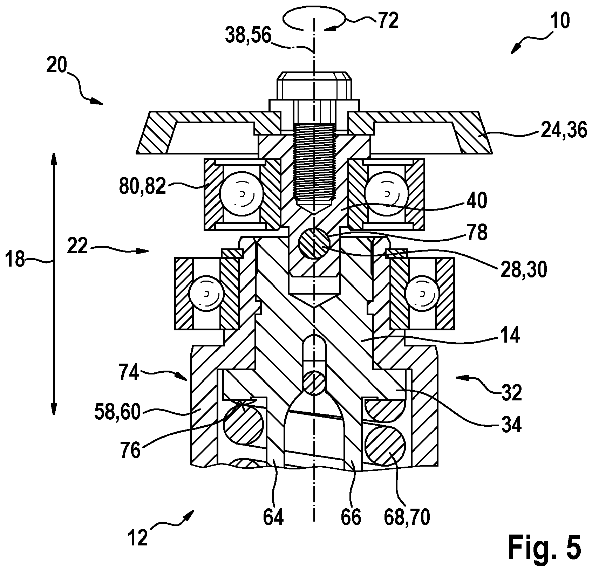

Des Weiteren weist die Spanneinheit 12 ein Federelement 68 auf, das dazu vorgesehen ist, das Spannelement 14 entlang der Axialrichtung 18 mit einer Federkraft zu beaufschlagen (Figur 5). Das Federelement 68 ist hierbei als Druckfeder 70 ausgebildet. Es ist jedoch auch denkbar, dass das Federelement 68 von einem anderen, einem Fachmann als sinnvoll erscheinenden Federelement gebildet wird, wie beispielsweise einer Zugfeder, einer Tellerfeder usw. Ferner ist es ebenfalls denkbar, dass die Spanneinheit 12 mehr als ein Federelement 68 zur Beaufschlagung des Spannelements 14 mit einer Federkraft aufweist. Das Spannelement 14 erstreckt sich in einem montierten Zustand entlang der Axialrichtung 18 durch die Druckfeder 70 hindurch. Somit ist die Druckfeder 70 entlang einer Umfangsrichtung 72 zumindest um einen Teilbereich des Spannelements 14 angeordnet. Die Umfangsrichtung 72 verläuft in einer sich zumindest im Wesentlichen senkrecht zur Axialrichtung 18 Ebene. Die Druckfeder 70 stützt sich in einem montierten Zustand mit einem Ende 74 an einer Anlagefläche 76 des Spannelements 14 ab. Die Anlagefläche 76 ist hierbei kreisringförmig ausgebildet.

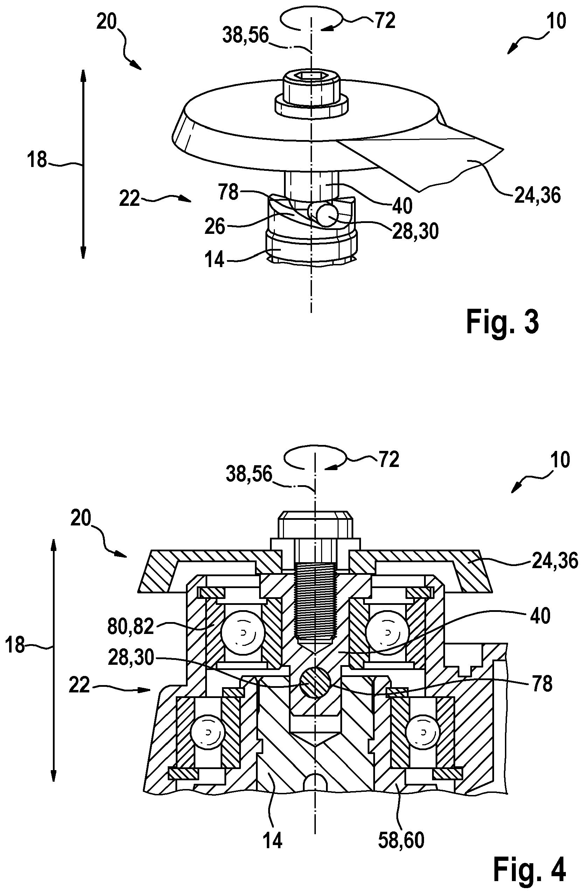

Die Werkzeugspannvorrichtung 10 umfasst ferner eine Übersetzungseinheit 22, die dazu vorgesehen ist, in Abhängigkeit zumindest einer Bewegungskomponente eines Bedienelements 24 der Bedieneinheit 20 ein Übersetzungsverhältnis zu verändern. Das Bedienelement 24 der Bedieneinheit 20 ist als Bedienhebel 36 ausgebildet, der schwenkbar um eine parallel zur Axialrichtung 18 verlaufende Schwenkachse 38 gelagert ist. Die Schwenkachse 38 verläuft hierbei koaxial zur Rotationsachse 56 der Hohlwelle 60. Ferner umfasst die Übersetzungseinheit 22 eine Steuerkurve 26, die, entlang eines Verlaufs der Steuerkurve 26 in zumindest zwei verschiedenen Punkten der Steuerkurve 26 betrachtet, zueinander verschiedene Steigungen in Axialrichtung 18 aufweist (Figur 3). Die Steuerkurve 26 ist an einer der Bedieneinheit 20 zugewandten Seite des Spannelements 14 angeordnet. Hierbei ist die Steuerkurve 26 einstückig mit dem Spannelement 14 ausgebildet. Die Übersetzungseinheit 22 weist ferner eine weitere Steuerkurve (hier nicht näher dargestellt) auf, die entlang der Umfangsrichtung 72 versetzt zur Steuerkurve 26 an dem Spannelement 14 angeordnet ist. Die weitere Steuerkurve weist hierbei eine zur Steuerkurve 26 analogen Verlauf auf. Somit weist die weitere Steuerkurve, entlang eines Verlaufs der weiteren Steuerkurve in zumindest zwei verschiedenen Punkten der weiteren Steuerkurve betrachtet, zueinander verschiedene Steigungen in Axialrichtung 18 auf.

Ferner weist die Übersetzungseinheit 22 ein Abtastelement 28 auf, das dazu vorgesehen ist, das Spannelement 14 in Abhängigkeit des Verlaufs der Steuerkurve 26 entlang der Axialrichtung 18 zu bewegen. Das Abtastelement 28 ist als ein Bolzen 30 ausgebildet. Der Bolzen 30 weist eine Längserstreckung auf, die in einem montierten Zustand entlang einer sich zumindest im Wesentlichen senkrecht zur Axialrichtung 18 erstreckenden Richtung verläuft. Der Bolzen 30 ist dazu vorgesehen, in zumindest einem Betriebsmodus jeweils mit zwei sich gegenüberliegenden Enden des Bolzens 30 mit der Steuerkurve 26 und der weiteren Steuerkurve in Kontakt gebracht zu werden. Die Bedieneinheit 20 umfasst ein Abtastaufnahmeelement 40 zur Aufnahme des Abtastelements 28 der Bedieneinheit 20, das drehfest mit dem Bedienhebel 36 verbunden ist. Das Abtastaufnahmeelement 40 erstreckt sich in einem montierten Zustand entlang der Axialrichtung 18. Ferner weist das Abtastaufnahmeelement 40 eine Ausnehmung 78 auf, die sich entlang einer zumindest im Wesentlichen senkrecht zur Axialrichtung 18 verlaufenden Richtung durch das Abtastaufnahmeelement 40 erstreckt. Die Ausnehmung 78 wird von einer Durchgangsbohrung gebildet, in der das Abtastelement 28 in einem montierten Zustand angeordnet ist. Hierbei entspricht ein Durchmesser der Ausnehmung 78 zumindest im Wesentlichen einer Abmessung des Abtastelements 28 entlang der Axialrichtung 18. Somit ist das Abtastelement 28 mittels einer Presspassung in der Ausnehmung 78 gehalten. Es ist jedoch auch denkbar, dass das Abtastelement 28 auf eine andere, einem Fachmann als sinnvoll erscheinenden Art und Weise in der Ausnehmung 78 gehalten wird. Ferner ist es jedoch auch denkbar, dass das Abtastelement 28 einstückig mit dem Abtastaufnahmeelement 40 ausgebildet ist und sich entlang einer zumindest im Wesentlichen senkrecht zur Axialrichtung 18 verlaufenden Richtung an zumindest zwei Stellen von dem Abtastaufnahmeelement 40 weg erstreckt. Das Abtastaufnahmeelement 40 ist ferner dazu vorgesehen, eine Lagerfunktion des Bedienhebels 36 im Werkzeugmaschinengehäuse 44 zu erfüllen (Figur 4). Hierbei ist ein als Kugellager 80 ausgebildetes Lagerelement 82 an dem Abtastaufnahmeelement 40 angeordnet. Das Kugellager 80 umschließt das Abtastaufnahmeelement 40 in einem Teilbereich des Abtastaufnahmeelements 40 entlang der Umfangsrichtung 72.

Die Werkzeugspannvorrichtung 10 weist ferner eine Entkopplungseinheit 32 auf, die dazu vorgesehen ist, die Bedieneinheit 20 in zumindest einem Betriebsmodus von einer Bewegung des Spannelements 14 zu entkoppeln. Die Entkopplungseinheit 32 ist dazu vorgesehen, die Bedieneinheit 20 in zumindest einem Betriebsmodus von einer oszillierenden Bewegung des Spannelements 14 um die Rotationsachse 56 zu entkoppeln. Hierbei weist die Entkopplungseinheit 32 ein Anschlagelement 34 auf, das dazu vorgesehen ist, eine Bewegung des Spannelements 14 entlang der Axialrichtung 18 in Richtung der Bedieneinheit 20 zu begrenzen. Das Anschlagelement 34 liegt hierbei in einem Betriebsmodus entlang der Axialrichtung 18 an einem Vorsprung der Hohlwelle 60 an. Die an dem Spannelement 14 angeordnete Steuerkurve 26 und die weitere Steuerkurve sind somit in einem Betriebsmodus entlang der Axialrichtung 18 beabstandet zum Abtastelement 28 angeordnet.

Zur Montage des Bearbeitungswerkzeugs 16 an der Werkzeugaufnahme 62 wird der Bedienhebel 36, ausgehend von einer am Werkzeugmaschinengehäuse 44 anliegenden Position des Bedienhebels 36, von dem Bediener in eine von dem Werkzeugmaschinengehäuse 44 weg gerichtete Richtung bewegt und somit um die Schwenkachse 38 gedreht. Hierbei wird das Abtastelement 28 zuerst in Richtung der Steuerkurve 26 und der weiteren Steuerkurve bewegt, bis Enden des als Bolzen 30 ausgebildeten Abtastelements 28 mit der Steuerkurve 26 und der weiteren Steuerkurve in Kontakt kommen. Bei einer weiteren Drehbewegung des Bedienhebels 36 um die Schwenkachse 38 gleitet das als Bolzen 30 ausgebildete Abtastelement 28 entlang der Steuerkurve 26 und der weiteren Steuerkurve. Hierdurch wird das Spannelement 14 entlang der Axialrichtung 18 in Richtung der Werkzeugaufnahme 62 bewegt. Die Spanneinheit 12 wird hierbei in einen Werkzeugwechselmodus überführt. Die Bewegung des Spannelements 14 entlang der Axialrichtung 18 in Richtung der Werkzeugaufnahme 62 ist hierbei abhängig von einem Verlauf der Steuerkurve 26 und der weiteren Steuerkurve und einem Öffnungswinkel des Bedienhebels 36. Der Öffnungswinkel wird bei einer Bewegung des Bedienhebels 36 um die Schwenkachse 38 ausgehend von der an dem Werkzeugmaschinengehäuse 44 anliegenden Position des Bedienhebels 36 von dem Bedienhebel 36 überstrichen.

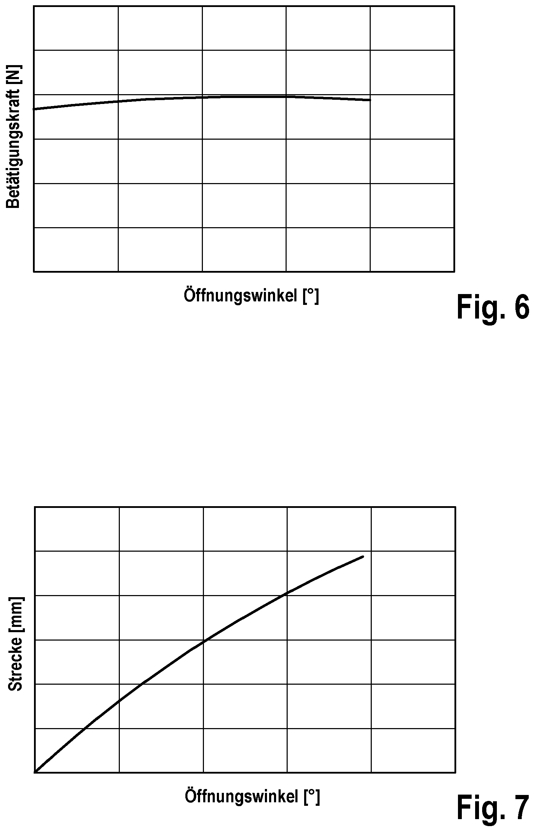

Figur 6 zeigt einen Zusammenhang zwischen dem vom Bedienhebel 36 überstrichenen Öffnungswinkel und einer von einem Bediener aufzubringenden Betätigungskraft zur Bewegung des Spannelements 14 entlang der Axialrichtung 18 in einem Diagramm. Das Diagramm zeigt einen unproportionalen Verlauf der Betätigungskraft zum Öffnungswinkel infolge des Verlaufs und/oder einer Geometrie der Steuerkurve 26 und der weiteren Steuerkurve. Somit ist auch eine vom Bediener aufzubringende Betätigungskraft zur Bewegung des Spannelements 14 entlang der Axialrichtung 18 infolge des Verlaufs und/oder der Geometrie der Steuerkurve 26 und der weiteren Steuerkurve unproportional zum Öffnungswinkel des Bedienhebels 36.

Figur 7 zeigt einen Zusammenhang zwischen einer vom Spannelement 14 zurückgelegten Strecke entlang der Axialrichtung 18 und dem vom Bedienhebel 36 überstrichenen Öffnungswinkel in einem weiteren Diagramm. Ein Verlauf der Strecke ist infolge des Verlaufs der Steuerkurve 26 und der weiteren Steuerkurve unproportional zum Öffnungswinkel. Das weitere Diagramm zeigt einen unproportionalen Verlauf der Strecke zum Öffnungswinkel infolge des Verlaufs und/oder der Geometrie der Steuerkurve 26 und der weiteren Steuerkurve. Somit ist auch eine bei einer Betätigung des Spannelements 14 mittels der Bedieneinheit 20 zurückgelegte Strecke des Spannelements 14 entlang der Axialrichtung 18 infolge des Verlaufs und/oder der Geometrie der Steuerkurve 26 und der weiteren Steuerkurve unproportional zum Öffnungswinkel des Bedienhebels 36.

## Patentansprüche

1. Werkzeugspannvorrichtung, insbesondere Oszillationswerkzeugspannvorrichtung, mit zumindest einer Spanneinheit (12), die zumindest ein Spannelement (14) zu einem Festspannen eines Bearbeitungswerkzeugs (16) in einer Axialrichtung (18) sowie zumindest eine Bedieneinheit (20) zur Betätigung des Spannelements (14) aufweist,
mit zumindest einer Übersetzungseinheit (22), die dazu vorgesehen ist, in Abhängigkeit zumindest einer Bewegungskomponente eines Bedienelements (24) der Bedieneinheit (20) ein Übersetzungsverhältnis zu verändern, **dadurch gekennzeichnet, dass** das Bedienelement (24) der Bedieneinheit (20) als Bedienhebel (36) ausgebildet ist, der schwenkbar um eine parallel zur Axialrichtung (18) verlaufende Schwenkachse (38) gelagert ist.

2. Werkzeugspannvorrichtung nach Anspruch 1,
**dadurch gekennzeichnet, dass** die Übersetzungseinheit (22) zumindest eine Steuerkurve (26) umfasst, die, entlang eines Verlaufs der Steuerkurve (26) in zumindest zwei verschiedenen Punkten der Steuerkurve (26) betrachtet, zueinander verschiedene Steigungen in Axialrichtung (18) aufweist.

3. Werkzeugspannvorrichtung nach einem der vorhergehenden Ansprüche,
**dadurch gekennzeichnet, dass** die Übersetzungseinheit (22) zumindest ein Abtastelement (28) aufweist, das dazu vorgesehen ist, das Spannelement (14) in Abhängigkeit des Verlaufs der Steuerkurve (26) entlang der Axialrichtung (18) zu bewegen.

4. Werkzeugspannvorrichtung nach Anspruch 3, **dadurch gekennzeichnet, dass** das Abtastelement (28) als Bolzen (30) ausgebildet ist.

5. Werkzeugspannvorrichtung nach Anspruch 4,
**dadurch gekennzeichnet, dass** der Bolzen (30) eine Längserstreckung aufweist, die in einem montierten Zustand entlang einer sich zumindest im Wesentlichen senkrecht zur Axialrichtung (18) erstreckenden Richtung verläuft.

6. Werkzeugspannvorrichtung nach einem der vorhergehenden Ansprüche, **gekennzeichnet durch** zumindest eine Entkopplungseinheit (32), die dazu vorgesehen ist, die Bedieneinheit (20) in zumindest einem Betriebsmodus von einer Bewegung des Spannelements (14) zu entkoppeln.

7. Werkzeugspannvorrichtung nach Anspruch 6,
**dadurch gekennzeichnet, dass** die Entkopplungseinheit (32) zumindest ein Anschlagelement (34) aufweist, das dazu vorgesehen ist, eine Bewegung des Spannelements (14) entlang der Axialrichtung (18) in Richtung der Bedieneinheit (20) zu begrenzen.

8. Werkzeugspannvorrichtung nach einem der vorhergehenden Ansprüche,
**dadurch gekennzeichnet, dass** die Bedieneinheit (20) zumindest ein Abtastaufnahmeelement (40) zur Aufnahme eines Abtastelements (28) der Übersetzungseinheit (22) umfasst, das zumindest drehfest mit dem Bedienhebel (36) verbunden ist.

9. Tragbare Werkzeugmaschine, insbesondere tragbare Werkzeugmaschine mit einer oszillierend antreibbaren Spindel, mit zumindest einer Werkzeugspannvorrichtung nach einem der vorhergehenden Ansprüche.

## Claims

1. Tool clamping fixture, in particular oscillation tool clamping fixture, having at least one clamping unit (12), which has at least one clamping element (14) for clamping a machining tool (16) in an axial direction (18), as well as at least one control unit (20) for actuating the clamping element (14),
having at least one conversion unit (22), which is provided to alter a conversion ratio in dependence on at least one motional component of a control element (24) of the control unit (20), **characterized in that** the control element (24) of the control unit (20) is configured as a control lever (36), which is mounted pivotably about a pivot axis (38) running parallel to the axial direction (18).

2. Tool clamping fixture according to Claim 1,
**characterized in that** the conversion unit (22) comprises at least one control cam (26), which, viewed along a course of the control cam (26) at at least two different points on the control cam (26), has mutually differing pitches in the axial direction (18).

3. Tool clamping fixture according to one of the preceding claims,
**characterized in that** the conversion unit (22) has at least one scanning element (28), which is provided to move the clamping element (14) in dependence on the course of the control cam (26) along the axial direction (18) .

4. Tool clamping fixture according to Claim 3,
**characterized in that** the scanning element (28) is configured as a bolt (30).

5. Tool clamping fixture according to Claim 4,
**characterized in that** the bolt (30) has a longitudinal extent which, in a mounted state, runs along a direction extending at least substantially perpendicular to the axial direction (18).

6. Tool clamping fixture according to one of the preceding claims,
**characterized by** at least one decoupling element (32), which is provided to decouple the control unit (20) in at least one operating mode from a motion of the clamping element (14).

7. Tool clamping fixture according to Claim 6,
**characterized in that** the decoupling unit (32) has at least one stop element (34), which is provided to limit a motion of the clamping element (14) along the axial direction (18) in the direction of the control unit (20).

8. Tool clamping fixture according to one of the preceding claims,
**characterized in that** the control unit (20) comprises at least one scanner receiving element (40) for receiving a scanning element (28) of the conversion unit (22), which scanner receiving element is connected at least in a rotationally fixed manner to the control lever (36).

9. Portable machine tool, in particular portable machine tool having an oscillatingly drivable spindle, having at least one tool clamping fixture according to one of the preceding claims.

## Revendications

1. Dispositif de serrage d'outil, en particulier dispositif de serrage d'outil oscillant, avec au moins une unité de serrage (12), qui présente au moins un élément de serrage (14) pour le serrage d'un outil d'usinage (16) dans une direction axiale (18) ainsi qu'au moins une unité de commande (20) pour actionner l'élément de serrage (14), avec au moins une unité de transmission (22), qui est prévue pour changer un rapport de transmission en fonction d'au moins une composante de mouvement d'un élément de commande (24) de l'unité de commande (20), **caractérisé en ce que** l'élément de commande (24) de l'unité de commande (20) est réalisé sous la forme d'un levier de commande (36), qui est monté de façon pivotante autour d'un axe de pivotement (38) orienté parallèlement à la direction axiale (18).

2. Dispositif de serrage d'outil selon la revendication 1, **caractérisé en ce que** l'unité de transmission (22) comprend au moins une came de commande (26) qui, considérée le long d'un tracé de la came de commande (26) en au moins deux points différents de la came de commande (26), présente des pentes différentes l'une de l'autre en direction axiale (18).

3. Dispositif de serrage d'outil selon l'une quelconque des revendications précédentes, **caractérisé en ce que** l'unité de transmission (22) présente au moins un élément palpeur (28), qui est prévu pour déplacer l'élément de serrage (14) le long de la direction axiale (18) en fonction du tracé de la came de commande (26).

4. Dispositif de serrage d'outil selon la revendication 3, **caractérisé en ce que** l'élément palpeur (28) est formé par un pivot (30).

5. Dispositif de serrage d'outil selon la revendication 4, **caractérisé en ce que** le pivot (30) présente une extension longitudinale, qui dans un état monté s'étend le long d'une direction orientée au moins essentiellement perpendiculairement à la direction axiale (18).

6. Dispositif de serrage d'outil selon l'une quelconque des revendications précédentes, **caractérisé par** au moins une unité de découplage (32), qui est prévue pour découpler l'unité de commande (20) d'un mouvement de l'élément de serrage (14) au moins dans un mode de fonctionnement.

7. Dispositif de serrage d'outil selon la revendication 6, **caractérisé en ce que** l'unité de découplage (32) présente au moins un élément de butée (34), qui est prévu pour limiter un mouvement de l'élément de serrage (14) le long de la direction axiale (18) en direction de l'unité de commande (20).

8. Dispositif de serrage d'outil selon l'une quelconque des revendications précédentes, **caractérisé en ce que** l'unité de commande (20) comprend au moins un élément de logement de balayage (40) destiné à recevoir un élément palpeur (28) de l'unité de transmission (22), qui est relié au moins sans rotation au levier de commande (36).

9. Machine-outil portable, en particulier machine-outil portable avec une broche pouvant être entraînée de façon oscillante, avec au moins un dispositif de serrage d'outil selon l'une quelconque des revendications précédentes.
